# EUROPEAN PATENT APPLICATION

(11) **EP 1 253 427 A2**
(43) Date of publication of application: **30.10.2002**
(21) Application number: 02009271.4
(22) Date of filing: 26.04.2002
(51) Int. Cl.: G01N 33/53

(54) **Bio-device and quantitative measurement apparatus and method using the same**

(30) Priority: 27.04.2001 JP 2001131410
(71) Applicant: MATSUSHITA ELECTRIC INDUSTRIAL CO., LTD., Kadoma-shi, Osaka 571-8501 (JP)
(72) Inventor: Kitawaki, Fumihisa, Osaka 571-0064 (JP); Shigeto, Nobuyuki, Kyotanabe-shi, Kyoto 610-0354 (JP); Kawamura, Tatsuro, Kyotanabe-shi, Kyoto 610-0351 (JP); Nadaoka, Masataka, Iyo-shi, Ehime 799-3113 (JP); Tanaka, Hirotaka, Matsuyama-shi, Ehime 791-1102 (JP); Takahashi, Mie, Niihama-shi, Ehime 792-0026 (JP)
(74) Representative: Schwabe - Sandmair - Marx

(57) **Abstract**

A bio-device includes a sample application section; an indicator substance holding section; and a determination section. The sample application section, the indicator substance holding section, and the determination section are located so that a liquid sample applied to the sample application section is transferred to the determination section via the indicator substance holding section. At least the indicator substance holding section and the determination section are included in a single member. The indicator substance holding section has a first substance group containing a substance specifically reacting with a target substance, wherein the first substance group is held so as to be capable of being eluted by the applied liquid sample. After the first substance group is eluted by the liquid sample applied to the sample application section, the first substance group flows as a mass having a leading end and a trailing end during flowing.

## Description

### BACKGROUND OF THE INVENTION

### 1. FIELD OF THE INVENTION:

The present invention relates to a bio-device, used in a dry chemistry examination method, for measuring a substance contained in a sample solution, and a quantitative measurement apparatus and method using such a bio-device.

### 2. DESCRIPTION OF THE RELATED ART:

Recently, a variety of test methods have been utilized in clinical tests. One of them is a test method using dry chemistry. Dry chemistry is a method for measuring a target substance in a liquid sample by dropping the liquid sample onto a reagent stored in a dry state in a solid layer matrix, such as, for example, a film or a test paper. This method can be carried out by a monolayer device or a multi-layer device. The monolayer device includes a single layer matrix including a filter holding a reagent. The multi-layer device includes a combination of a capillary-flow (developing) layer, a reaction layer, a reagent layer, and the like for holding a reagent. Both the monolayer and multi-layer devices, in which a reagent is already held on a solid layer matrix, have features in that (i) it is not necessary to adjust the reagent, (ii) the device is stored in a small space, and (iii) only a small amount of target substance is required. A representative test method using dry chemistry is immunochromatography. Immunochromatography is a test method which utilizes an antigen-antibody reaction and a capillaryphenomenon. In a device for immunochromatography, an immobilized antibody (or an antigen) and an antibody (or an antigen) labeled as an indicator reagent are held in a dry state on a carrier formed of a porous member represented by a membrane filter. In a test, a test sample containing an antigen (or an antibody) is placed on the device and flows by a capillary phenomenon. Reaction sites are colored by sandwich-type antigen-antibody reactions, so as to identify an antigen (or an antibody), detect the presence or absence thereof, or measure the amount thereof. In addition to the sandwich type reaction, a competitive type reaction may be used as an alternative antigen-antibody reaction for immunochromatography. The structure of a device and the test method are substantially the same as described above.

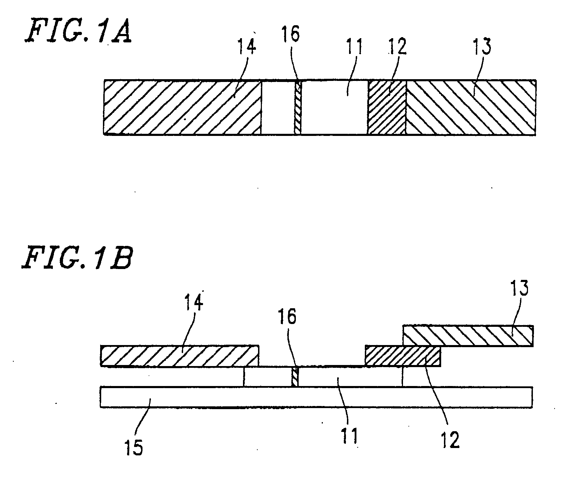

Figures **1A** and **1B** show a structure of a conventional immunochromatographic device. Figure **1A** is a plan view thereof, and Figure **1B** is a side view thereof. The conventional immunochromatographic device includes a substrate **11** formed of a porous material. A determination section **16** is provided in the substrate **11**. A sample application section **13**, an indicator substance holding section **12**, a water absorption section **14** which are formed of different materials are provided on the substrate **11**. The substrate **11**, the sample application section **13**, the indicator substance holding section **12**, and the water absorption section **14** are stacked on an underlying substrate **15**.

The indicator substance holding section **12** carries a first antibody specifically reacting with a target substance and labeled with a labeling substance, in a state where the first antibody can be eluted. The determination section **16** has a second antibody specifically reacting with the target substance immobilized thereto. A liquid sample applied to the sample application section **13** flows while eluting the first antibody from the indicator substance holding section **12** and reaches the determination section **16.** When the target substance is contained in the liquid sample, the first antibody - target substance - second antibody complex is formed in the determination section **16.** Since the indicator substance holding section **12** and the determination section **16** are formed of different materials, the first antibody from the indicator substance holding section **12** is diffused as the first antibody gradually exudes from the indicator substance holding section **12** toward the determination section **16**. Therefore, the flow of the first antibody from the indicator substance holding section **12** is not disrupted.

In addition to the above-described advantages of dry chemistry, a test method utilizing immunochromatography has the advantages of ease of handling, quick determination, and low cost. The test method is applicable to point of care testing (POCT) which has recently received attention, as well as clinical tests. POCT is a general term for clinical tests for which the time period from the stage of sampling to the stage of obtaining the result is considered to be most important.

Conventional bio-devices usable for POCT require 3 to 5 minutes to obtain the determination result after sampling. There has been a demand for a reduction of this time period. For some target substances, quantitative measurement is often required in addition to qualitative measurement. The conventional bio-devices usable for POCT do not provide satisfactory quantitative measurement in terms of reproducibility.

### SUMMARY OF THE INVENTION

According to one aspect of the invention, a bio-device used for measuring a target substance included in a liquid sample includes a sample application section; an indicator substance holding section; and a determination section. The sample application section, the indicator substance holding section, and the determination section are located so that the liquid sample applied to the sample application section is transferred to the determination section via the indicator substance holding section. At least the indicator substance holding section and the determination section are included in a single member. The indicator substance holding section has a first substance group containing a substance specifically reacting with the target substance, wherein the first substance group is held so as to be capable of being eluted by the applied liquid sample. After the first substance group is eluted by the liquid sample applied to the sample application section, the first substance group flows as a mass having a leading end and a trailing end during flowing.

According to another aspect of the invention, a bio-device used for measuring a target substance included in a liquid sample includes a sample application section; an indicator substance holding section; and a determination section. The sample application section, the indicator substance holding section, and the determination section are located so that the liquid sample applied to the sample application section is transferred to the determination section via the indicator substance holding section. At least the indicator substance holding section and the determination section are included in a single member. The indicator substance holding section has a first substance group containing a substance specifically reacting with the target substance, wherein the first substance group is held so as to be capable of being eluted by the applied liquid sample. The determination section has a second substance group containing a substance specifically reacting with the target substance, the second substance group being in an immobilized state. In a process in which the first substance group contained in the indicator substance holding section is eluted by the action of the liquid sample applied to the sample application section and reaches the determination section together with the liquid sample while being diffused in a moving direction of the liquid sample, a holding width A of the indicator substance holding section in the moving direction of the liquid sample before the application of the liquid sample, and a diffusion width B, which is a width of the indicator substance, relative to the holding width A, in the moving direction of the liquid sample when a trailing end of the first substance group reaches the determination section, has a ratio A:B of 1:0.25 to 1:1.

In one embodiment of the invention, an area between the indicator substance holding section and the determination section is equal to or greater than 3 mm² and equal to or less than 150 mm².

In one embodiment of the invention, the sample application section, the indicator substance holding section, and the determination section are in a dry state before the liquid sample is applied.

In one embodiment of the invention, the liquid sample is a bodily fluid.

In one embodiment of the invention, the substance contained in the first substance group specifically reacting with the target substance is labeled with a coloring substance, a fluorescent substance, a phosphorescent substance, a light-emitting substance, an oxidoreductant, an enzyme, a nucleic acid, or an endoplasmic reticulum.

In one embodiment of the invention, the coloring substance is a gold colloidal particle.

In one embodiment of the invention, the first substance group includes a first antibody against the target substance, and the second substance group includes a second antibody against the target substance.

In one embodiment of the invention, the first substance group includes a first antibody and a second antibody against the target substance, the second antibody is labeled with biotin, and the second substance group includes avidin specifically reacting with biotin.

In one embodiment of the invention, the first substance group includes a first antibody and a second antibody against the target substance, the second antibody is labeled with a magnetic substance, and the second substance group includes a substance magnetically capturing the magnetic substance.

In one embodiment of the invention, the indicator substance holding section and the determination section are included in a porous member.

In one embodiment of the invention, the porous member is a nitrocellulose-based membrane.

In one embodiment of the invention, the sample application section is stacked on the porous member including the indicator substance holding section and the determination section.

In one embodiment of the invention, the bio-device further includes a water absorption section provided on the porous member including the indicator substance holding section and the determination section, the water absorption section being opposite to the indicator substance holding section with respect to the determination section.

In one embodiment of the invention, the bio-device further includes a member, which is non-permeable to the liquid sample, adhering to at least a portion of the sample application section, the indicator substance holding section and the determination section.

According to still another aspect of the invention, a quantitative measurement apparatus for measuring a target substance included in a liquid sample includes any of the above-described bio-devices; and a measuring device for quantitatively measuring a physical or chemical signal obtained at a determination section of the bio-device.

According to still another aspect of the invention, a quantitative measurement method for measuring a target substance included in a target substance using the above-described quantitative measurement apparatus includes the steps of applying a prescribed amount of a liquid sample to a sample application section; and quantitatively measuring a physical or chemical signal obtained at a determination section by a measuring device of the quantitative measurement apparatus.

Thus, the invention described herein makes possible the advantages of providing a bio-device for realizing rapid, highly precise, and highly reproducible qualitative and quantitative measurements of a target substance in a liquid sample; and a quantitative measurement apparatus and a quantitative measurement apparatus method using such a bio-device.

These and other advantages of the present invention will become apparent to those skilled in the art upon reading and understanding the following detailed description with reference to the accompanying figures.

### BRIEF DESCRIPTION OF THE DRAWINGS

Figure **1A** is a plan view of a conventional immunochromatographic device;
Figure **1B** is a side view of the conventional immunochromatographic device shown in Figure **1A**;
Figure **2** is a graph illustrating an exemplary method for determining a holding width A of a bio-device according to the present invention using an optical method;
Figure **3** is a graph illustrating an exemplary method for determining a diffusion width B of the bio-device according to the present invention using an optical method;
Figure **4** is an isometric view of a position setting device body **40** for determining the determination section according to the present invention;
Figure **5A** is a plan view of a bio-device according to an example of the present invention;
Figure **5B** is a side view of the bio-device shown in Figure **5A**;
Figure **6** is a graph illustrating the relationship among the diffusion width B/holding width A, the CV value and the absorbance obtained when the hCG concentration in urine was measured using the bio-device according to the present invention;
Figure **7** is a graph illustrating the hCG concentration in urine obtained using a bio-device having the diffusion width B/holding width A ratio of 5%;
Figure **8** is a graph illustrating the hCG concentration in urine obtained using a bio-device having the diffusion width B/holding width A ratio of 50%;
Figure **9** is a graph illustrating the hCG concentration in urine obtained using a bio-device having the diffusion width B/holding width A ratio of 120%;
Figure **10** is a graph illustrating the hCG concentration in urine obtained using a bio-device in which the area between an indicator substance holding section and a determination section is 175 mm²;
Figure **11** is a graph illustrating the hCG concentration in urine obtained using a bio-device in which the area between an indicator substance holding section and a determination section is 50 mm²; and
Figure **12** is a graph illustrating the hCG concentration in urine obtained using a bio-device in which the area between an indicator substance holding section and a determination section is 5 mm².

### DESCRIPTION OF THE PREFERRED EMBODIMENTS

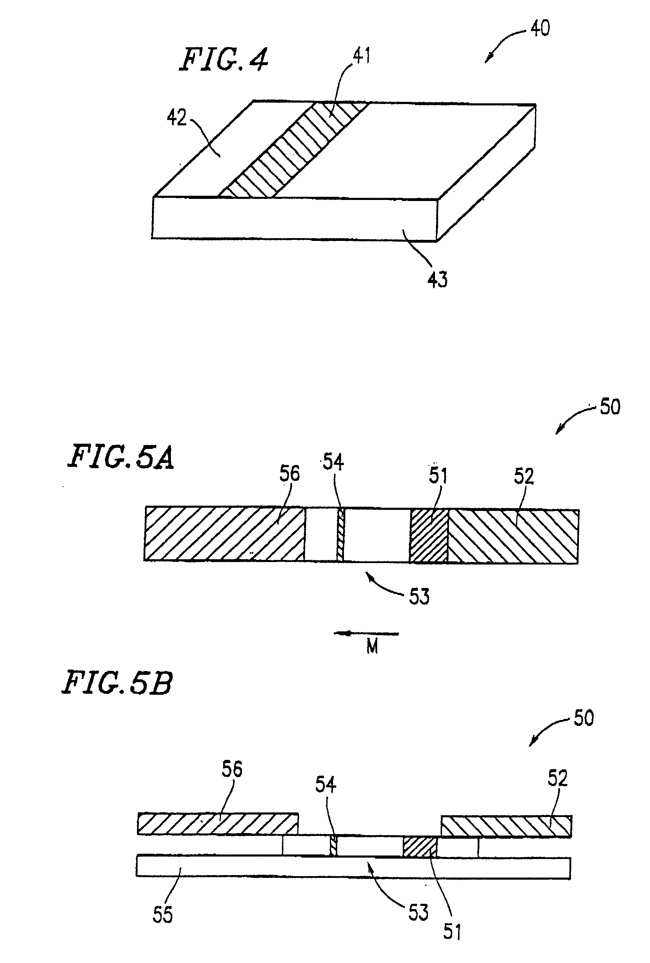

A bio-device according to an example of the present invention used for measuring a target substance contained in a liquid sample will be described with the reference numerals shown in Figures **5A** and **5B.**

According to the present invention, the bio-device includes a sample application section **52;** an indicator substance holding section **51**; and a determination section **54**. The sample application section **52,** the indicator substance holding section **51,** and the determination section **54** are located so that the liquid sample applied to the sample application section **52** is transferred to the determination section **54** via the indicator substance holding section **51**. At least the indicator substance holding section **51** and the determination section **54** are included in a single member. The indicator substance holding section **51** has a first substance group containing a substance specifically reacting with the target substance with an indicator. The substance group is held so as to be capable of being eluted.

After the first substance group is eluted by the liquid sample applied to the sample application section **52**, the first substance group flows as a mass having a leading end and a trailing end during flowing.

More specifically, the first substance group contained in the indicator substance holding section **51** is eluted by the action of the liquid sample which is applied to the sample application section **52**, and reaches the determination section **54** together with the liquid sample while being diffused in a moving direction of the liquid sample (direction of arrow M in Figure **5A**).

The determination section **54** has a second substance group containing a substance specifically reacting with the target substance. The second substance group is immobilized to the determination section **54**.

In this specification, the term "holding width A" is defined as the width of an indicator substance holding section formed of the first substance group, in a moving direction of the liquid sample, before the application of the liquid sample. The term "diffusion width B" is defined as a width of the first substance group, relative to the holding width A, in the moving direction of the liquid sample when a trailing end of the first substance group reaches the determination section. According to the present invention, the holding width A and the diffusion width B has a ratio A:B of 1:0.25 to 1:1. Also in this specification, the term "moving direction" is defined as the moving direction of the liquid sample represented by arrow **M** in Figure **5A**.

In the bio-device according to the present invention, the indicator substance holding section **51** and the determination section **54** are included in a single member. Due to such a structure, when a liquid sample is introduced to the sample application section **52**, the first substance group, eluted by the action of the liquid sample, starts moving in a state of a mass and moves toward the determination section **54** while being diffused in the moving direction. Then, the first substance group passes through the determination section **54** in a short period of time. In a conventional bio-device, by contrast, the first antibody (corresponding to the first substance group) from the indicator substance holding section **12** is diffused toward the determination section **16** as the first antibody gradually exudes without disruption. Therefore, a longer time period is required until a sufficient amount of first antibody reaches the determination section **16** than in the bio-device according to the present invention. The bio-device according to the present invention, which causes the reaction at the determination section **54** in a shorter time period than in the conventional bio-device, shortens the determination time. For example, most of the pregnancy tests performed by immunochromatographic devices require 3 to 5 minutes to obtain a sufficient determination result after sampling. The bio-device according to the present invention provides a determination result in a shorter time period than 3 minutes with certainty.

When the first substance group starts eluting from the indicator substance holding section **51**, a width of the first substance group in the moving direction with respect to the holding width A is smaller than 25%. As the first substance group comes closer to the determination section **54**, the width of the first substance group increases.

The width of the first substance group increases for the following reason. The flowing speed of the liquid sample which moves the first substance group is maximum when the liquid sample is applied to the sample application section **52**, and decreases overtime. As the flowing speed of the liquid sample decreases, the first substance group is diffused more and more easily. Therefore, the width of the first substance group increases overtime.

In order to improve the precision of the quantitative measurement of a target substance contained in a liquid sample using the bio-device according to the present invention, it is required that the reaction at the determination section **54** occurs uniformly. As the width of the first substance group increases, the diffused state of the first substance group becomes less uniform among various points of the first substance group, due to the moving speed difference. Accordingly, the precision of the quantitative measurement depends on the magnitude of the diffusion width B.

One exemplary index showing the precision of the quantitative measurement is coefficient of variation (hereinafter, referred to as a "CV value", which is calculated as a standard deviation/average × 100). As the CV value is smaller, the precision of measurement is higher.

In the bio-device in this example, the determination section **54** is located such that the ratio of A:B is in the range of 1:0.25 to 1:1; i.e., the B/A ratio is in the range of 25% to 100%. Due to such a setting, a measurement which is more precise than in a conventional device can be realized in a short period of time and with higher reproducibility.

In the case where the determination section **54** is provided at such a position that the B/A ratio is less than 25%, the target substance - first substance group complex passes through the determination section **54** in an excessively short time period after the liquid sample is applied to the sample application section **52**. Therefore, the target substance - first substance group complex passes through the determination section **54** at a very high speed, before sufficiently reacting with the second substance group at the determination section **54**. Therefore, sufficient determination results cannot be obtained.

In the case where the determination section **54** is provided at such a position that the B/A ratio is more than 100%, an excessively long time period is required before the target substance - first substance group complex passes through the determination section **54**. The flowing speed of the liquid sample is low and thus rapid determination cannot be provided, although the target substance - first substance group complex reacts with the second substance group sufficiently. Additionally, the diffusion width B is large and is not uniform, and therefore the precision of quantitative measurement is deteriorated.

In the case where the liquid sample has a relatively high viscosity such as, for example, blood, the flowing speed of the liquid sample is relatively low. Therefore, the ratio of A:B is preferably in the range of 1:0.25 to 1:0.50, so as to shorten the time period before the target substance - first substance group complex passes through the determination section **54**. By contrast, in the case where the liquid sample has a relatively low viscosity such as, for example, urine, the flowing speed of the liquid sample is relatively high. Therefore, the ratio of A:B is preferably in the range of 1:0.50 to 1:1, so as to extend the time period before the target substance - first substance group complex passes through the determination section **54.**

The holding width A and the diffusion width B can be determined by, for example, an optical method. First, an exemplary method for determining the holding width A will be described.

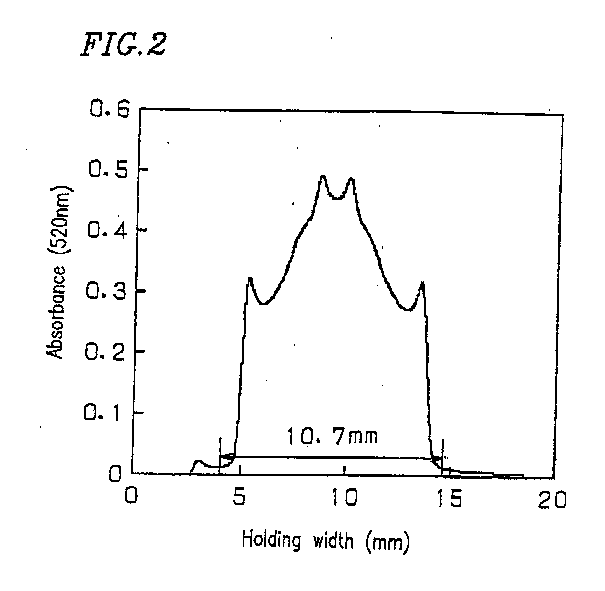

A bio-device is placed on a scanning stage of a reflection absorbance spectrometer. The bio-device is scanned so as to measure the reflection absorbance of the first substance group. Figure **2** is a graph illustrating the resulting relationship between the holding width A and the absorbance. Based on Figure **2,** the holding width A is defined as the scanning length in the range where the absorbance is equal to or greater than 0.01.

The diffusion width B (i.e., a width of the first substance group, relative to the holding width A, in the moving direction when a trailing end of the first substance group reaches the determination section **54**) can be determined as follows. The manner of the flow of the first substance group on the bio-device occurring when the liquid sample is applied to the sample application section **52** is examined by measurement over time of reflection absorbance. The light is pre-set to be directed to a position of the bio-device which is appropriate for the determination section **54.** Then, the liquid sample is dropped to the bio-device so as to cause the first substance group to flow. When the first substance group reaches the position irradiated with light, the absorbance drastically increases. When the first substance group further moves and passes over the position irradiated with light, the absorbance decreases. Based on the difference in absorbance, the leading end and the trailing end of the first substance group are found. Thus, the width of the first substance group when the trailing end thereof reaches the determination section **54** can be obtained. Thus, the diffusion width B can be obtained. In this example, the absorbance is measured using a portion of the bio-device which does not contain the second substance group as the reference. The point at which the absorbance decreases to 0.3 after first increasing is defined as the trailing end.

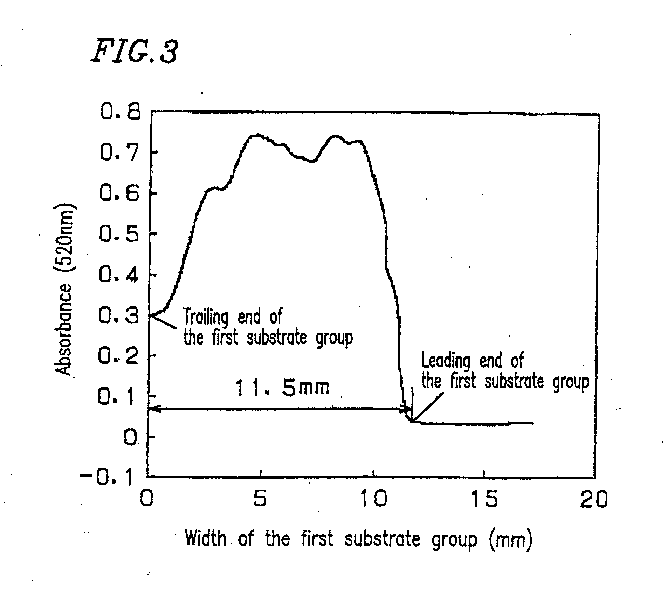

When the position of the trailing end of the first substance group is obtained, the bio-device is scanned in a direction opposite to the moving direction of the liquid sample (a direction opposite to the direction represented by arrow **M** in Figure **5A**). Thus, as shown in Figure **3,** the reflection absorbance is obtained with respect to the scanning distance. Based on Figure **3**, a point, at which the absorbance is substantially stabilized after first increasing and then decreasing when the bio-device is scanned in the opposite direction from the trailing end of the first substance group, is defined as the leading end. The width of the first substance group in the moving direction is the distance between the leading end and the trailing end of the first substance group.

The first substance group may be non-uniform depending on the manner of the flow of the liquid sample or the state of the surface of the bio-device. Tailing may occur to the trailing end of the first substance group. The reason why the point at which the absorbance decreases to 0.3 is defined as the trailing end as described above is to eliminate the tail portion from the diffusion width B even when tailing occurs.

The holding width A and the diffusion width B can be determined by other methods than the optical method. For example, when the indicator substance contained in the first substance group has electrochemical properties, the holding width A and the diffusion width B can be determined by using an electrochemical detection method instead of a method using a reflection absorbance spectrometer.

According to another embodiment of the invention, the bio-device includes a sample application section **52**; an indicator substance holding section **51**; and a determination section **54.** The sample application section **52**, the indicator substance holding section **51,** and the determination section **54** are located so that the liquid sample applied to the sample application section **52** is transferred to the determination section **54** via the indicator substance holding section **51**. At least the indicator substance holding section **51** and the determination section **54** are included in a single member. The indicator substance holding section **51** has a first substance group containing a substance specifically reacting with the target substance In this state, the substance group can be eluted. The determination section **54** has a second substance group specifically reacting with the target substance. The second substance group is immobilized to the determination section **54**.

The area between the indicator substance holding section **51** and the determination section **54** is in the range of 3 mm² and 150 mm².

With such a structure, the time period from when the liquid sample is applied to the sample application section **52** until the first substance group passes through the determination section **54** can be controlled. Therefore, the diffusion width B is uniformized. Thus, the same effect as provided by the bio-device in the above-described embodiment can be provided.

In the case where the liquid sample has a relatively high viscosity such as, for example, blood, the flowing speed of the liquid sample is relatively low. Therefore, the area between the indicator substance holding section **51** and the determination section **54** is preferably in the range of 3 mm² to 25 mm², so as to shorten the time period before the first substance group passes through the determination section **54**. By contrast, in the case where the liquid sample has a relatively low viscosity such as, for example, urine, the flowing speed of the liquid sample is relatively high. Therefore, the area between the indicator substance holding section **51** and the determination section **54** is preferably in the range of 25 mm² to 150 mm², so as to extend the time period before the first substance group passes through the determination section **54.**

According to the present invention, the sample application section **52,** the indicator substance holding section **51** and the determination section **54** are preferably in a dry state before a liquid sample is applied to the sample application section **52.** Before the liquid sample is applied, the bio-device can be provided as a dry device, which is easier to handle and is suitable as a simple device usable for POCT.

The bio-device according to the present invention is usable in the fields of, for example, urine tests, pregnancy tests, water quality tests, stool tests, soil analysis, and food analysis. A liquid sample may be an aqueous solution or an organic solution. Solutions usable as the liquid sample include, for example, bodily fluids, river water, seawater, groundwater, and aqueous solutions obtained by dissolving soil or food. The bodily fluids include, for example, blood, plasma, serum, urine, saliva, sweat, and tears.

Target substances in the present invention include, for example, bacteria, erythrocyte, proteins and viruses. Exemplary bacteria include tubercle bacillus, and Enterobacteriaceae. Exemplary proteins include hemoglobin, hemoglobin A1c, high density lipoproteins (HDL), low density lipoproteins (LDL), C-reactive proteins (CRP), albumin, and α fetoproteins. Exemplary viruses include AIDS virus and hepatitis C virus.

The substance, contained in the first substance group, specifically reacting with the target substance is preferably labeled with a coloring substance, a fluorescent substance, a phosphorescent substance, a light-emitting substance, an oxidoreductant, an enzyme, a nucleic acid, or an endoplasmic reticulum. Exemplary coloring substances include gold colloid, silver colloid, selenium colloid, colored latex, cyanine, and azo. Exemplary fluorescent substances include aromatic compounds such as pyrene, aromatic compounds substituted with functional groups such as dansyl, fluorescein, rhodamine, and coumarin. Exemplary phosphorescent substances include benzophenone. Exemplary light-emitting substances include substances emitting light by the format of, for example, a light emitting reaction of luciferin and ATP. Exemplary oxidoreductants include substances generating an electric current by the format of, for example, an oxidation-reduction reaction of glucose and glucose oxidase. Exemplary endoplasmic reticula include micelle and liposome. Among these substances, gold colloid is most preferable.

The substances specifically reacting with the target substance according to the present invention include, for example, antigens, antibodies, nucleic acids, enzymes and receptor. It is preferable that the first substance group includes a first antibody against the target substance and the second substance group includes a second antibody against the target substance. The first antibody and the second antibody may each be any antibody specifically reacting with the target substance. Exemplary antibodies usable as the first and second antibodies include anti-cell antibodies, anti-protein antibodies, anti-glycoprotein antibodies, anti-enzyme antibodies, anti-polysaccharide antibodies, anti-bacterium antibodies, and anti-virus antibodies. Either monoclonal antibodies or polyclonal antibodies are usable.

As the first and second antibodies specifically reacting with one same type of target substance, any combination of antibodies which recognize different antigenic determinants (epitopes) of the target substance is usable.

The first substance group may include a first antibody and a second antibody against the target substance, the second antibody may be labeled with biotin, and the second substance group may include avidin specifically reacting with biotin. Due to such a structure, the first antibody and the second antibody labeled with avidin flow while forming a complex via the target substance in the liquid sample. At the determination section **54**, biotin and avidin in the complex specifically react with each other, thus allowing the target substance to be captured at the determination section **54.**

The first substance group may include a first antibody and a second antibody against the target substance, the second antibody may be labeled with a magnetic substance, and the second substance group may include a substance magnetically capturing the magnetic substance. Due to such a structure, the first antibody and the second antibody labeled with the magnetic substance flow while forming a complex via the target substance in the liquid sample. At the determination section **54**, the magnetic substance in the complex is magnetically captured, thus allowing the target substance to be caught at the determination section **54.** The magnetic substance is, for example, amagnetic particle such as iron oxide or aluminum oxide. The magnetic substance can be magnetically captured by, for example, providing a magnet in the determination section **54**.

The sample application section **52**, the indicator substance holding section **51** and the determination section **54** may be formed of any material which allows the liquid sample to flow at an appropriate speed by a capillary phenomenon. For example, the sample application section **52**, the indicator substance holding section **51** and the determination section **54** may be formed of a porous member such as, for example, a nitrocellulose-based membrane, a cellulose acetate membrane, and a glass filter. Among these members, a nitrocellulose-based membrane is preferably used.

The sample application section **52** may be stacked on the porous member including the indicator substance holding section **51** and the determination section **54**. Due to such a structure, a sufficient amount of liquid sample can be supplied to the porous member including the indicator substance holding section **51** and the determination section **54**. The sample application section **52** may be formed of, for example, a water absorptive porous member formed of unwoven cloth or the like.

The bio-device may further include a water absorption section **56** provided on the porous member including the indicator substance holding section **51** and the determination section **54**, on the side opposite to the indicator substance holding section **51** with respect to the determination section **54**. Due to such a structure, the excessive amount of liquid sample on the porous member including the indicator substance holding section **51** and the determination section **54** can be absorbed. The water absorption section **56** may be formed of, for example, a porous member such as a glass fiber filter.

The bio-device is preferably accommodated in a hollow case. The hollow case is formed of, for example, a plastic material, and has openings in correspondence with at least the determination section **54** and the sample application section **52.** The hollow case provides an effect of preventing the liquid sample from leaking outside.

A member which is non-permeable to the liquid sample preferably adheres to at least a portion of the sample application section, the indicator substance holding section and the determination section. For example, an adhesive tape formed of a material which is non-permeable to the liquid sample may be bonded. This provides an effect of preventing the liquid sample from leaking outside, and also an effect of controlling the flow rate of the liquid sample on the bio-device and uniformizing the flow of the liquid sample.

A quantitative measurement apparatus according to the present invention is for measuring a target substance included in a liquid sample. The quantitative measurement apparatus includes the above-described bio-device; and a measuring device for quantitatively measuring a physical or chemical signal obtained at a determination section **54** of the bio-device. As the measuring device for quantitatively measuring a physical or chemical signal, any device which can convert a change in the color intensity of the determination section **54** into a numerical value is usable. For example, a device which can measure the reflection absorbance is usable.

A quantitative measurement method according to the present invention is for measuring a target substance included in a target substance using the above-described quantitative measurement apparatus. The quantitative measurement method comprising the steps of applying a prescribed amount of a liquid sample to a sample application section **52**; and quantitatively measuring a physical or chemical signal obtained at a determination section **54** by a measuring device of the quantitative measurement apparatus. In the step of applying the prescribed amount of the liquid sample to the sample application section **52,** the liquid sample is accurately measured using, for example, a pipet or the like and delivered to the sample application section **52**.

In the bio-device according to the present invention, the indicator substance holding section **51** and the determination section **54** are included in a single member. Due to such a structure, when a liquid sample is introduced to the sample application section **51**, the first substance group, eluted by the action of the liquid sample, starts moving in the state of amass andmoves toward the determination section **54** while being diffused in the moving direction. Then, the first substance group passes through the determination section **54** in a short period of time. In a conventional bio-device, by contrast, the first antibody (corresponding to the first substance group) from the indicator substance holding section **12** is diffused toward the determination section **16** as the first antibody gradually exudes without disruption. Therefore, a longer time period is required until a sufficient amount of first antibody reaches the determination section **16** than in the bio-device according to the present invention. The bio-device according to the present invention, which causes the reaction at the determination section **54** in a shorter time period than in the conventional bio-device, shortens the determination time. For example, most of the pregnancy tests performed by immunochromatographic devices require 3 to 5 minutes to obtain a sufficient determination result after sampling. The bio-device according to the present invention provides a determination result in a shorter time period than 3 minutes with certainty.

In a bio-device according to the present invention, the determination section **54** is located such that the ratio of A:B is in the range of 1:0.25 to 1:1; i.e., the B/A ratio is in the range of 25% to 100%. Due to such a setting, measurement which is more precise than in a conventional device can be realized in a short period of time and with higher reproducibility.

The present invention will be further explained by way of illustrative, but non-limiting examples with reference to drawings.

### (Example 1)

An exemplary bio-device for measuring hCG in urine will be described.

### (a) Preparation of a device for setting the position of a determination section

Figure **4** shows a structure of a position setting device body **40** for setting the position of a determination section. The position setting device body **40** includes a nitrocellulose membrane **43** having a sample application section **42** and an indicator substance holding section **41** thereon. The position setting device body **40** was prepared as follows.

Gold colloidal particles as a coloring substance were prepared as follows. A 1% aqueous solution of citric acid was added to a 0.01% aqueous solution of chloroauric acid having a temperature of 100°C, which was in a reflux state. The solution was continuously refluxed for 30 minutes and then left at room temperature to be cooled. The resultant gold colloidal solution was adjusted to be pH9 with a 0.2 M aqueous solution of potassium carbide. To the resultant solution, anti-hCG-α antibody was added and stirred for several minutes. Then, a 10% aqueous solution of BSA (bovine serum albumin) of pH9 was added thereto in such an amount as to provide a final concentration of 1%. Then, the mixture was stirred. Thus, an antibody-gold colloid complex (labeled antibody or the first substance group) as an indicator substance was obtained. The labeled antibody solution was centrifuged at 4°C for 50 minutes at 20,000 G so as to isolate the labeled antibody. The labeled antibody was suspended in a washing buffer solution (1% BSA-phosphate buffer). Then, the obtained substance was centrifuged so as to wash and isolate the labeled antibody. The labeled antibody was suspended in a washing buffer solution and filtered by a 0.8 µm filter. The amount of the labeled antibody was adjusted to 1/10 of the initial amount of the gold colloidal solution, and then the labeled antibody solution was stored at 4°C.

The labeled antibody solution was set in a solution injection apparatus, and applied to the nitrocellulose membrane **43** and dried. Thus, the position setting device body **40** (reaction layer carrier) having the indicator substance holding section **41**, which contains the labeled antibody (first substance group), on the nitrocellulose membrane **43** was produced. The position setting device body **40** was cut in a direction perpendicular to the indicator substance holding section **41** into a plurality of position setting devices each having a width of 0.5 cm.

### (b) Setting of the position of the determination section

A urine sample in an amount of 40 µl was dropped to the sample application section **42** of the resultant position setting device. The position setting device was set on a scanning stage of a reflection absorbance spectrometer. The manner of the flow of the labeled antibody on the position setting device after the liquid sample was applied was examined usingmeasurement over time of reflection absorbance. More specifically, light was directed to a position of the position setting device which was appropriate for the determination section. The holding width A of the indicator substance holding section **41** in the moving direction, and the diffusion width B were measured using the method described above. The light was directed to a plurality of other positions also appropriate for the determination section, and the holding width A and the diffusion width B were measured. Thus, the relationship between each of the positions appropriate for the determination section and the corresponding diffusion width B was obtained.

### (c) Preparation of a bio-device

Figures **5A** and **5B** show a bio-device **50** according to one example of the present invention. Figure **5A** is a plan view thereof, and Figure **5B** is a side view thereof. The bio-device **50** includes a support **55**; a nitrocellulose membrane **53,** having an indicator substance holding section **51** and a determination section **54**, provided on the support **55**; and a sample application section **52** and a water absorption section **56** provided on the nitrocellulose membrane **53**. The sample application section **52** is provided on a portion of the nitrocellulose membrane **53** which is in the vicinity of the indicator substance holding section **51**. The above portion of the nitrocellulose member **53** does not contain the indicator substance. The water absorption section **56** is provided on the opposite side of the nitrocellulose membrane **53** to the sample application section **52** with respect to the determination section **54**.

The bio-device **50** was prepared as follows.

First, the determination section **54** was provided. An aqueous solution of anti-hCG-β antibody having an appropriate concentration as a result of being diluted with a phosphate buffer was prepared. The antibody solution was applied on the nitrocellulose membrane **53** using a solution injection apparatus. Thus, the antibody was immobilized on the nitrocellulose membrane **53** as the determination section **54.** The resultant nitrocellulose membrane **53** with the determination section **54** was immersed in a Tris-HCl buffer solution containing 1% skim milk and mildly shaken for 30 minutes. The nitrocellulose membrane **53** was then put into a Tris-HCl buffer solution bath and mildly shaken for 10 minutes. Then, the nitrocellulose membrane **53** was again mildly shaken for 10 minutes in another Tris-HCl buffer solution bath. After being washed twice in this manner, the nitrocellulose membrane **53** was removed from the bath and dried at room temperature.

Next, the indicator substance holding section **51** was provided as follows.

Gold colloidal particles as a coloring substance were prepared as follows. A 1% aqueous solution of citric acid was added to a 0.01% aqueous solution of chloroauric acid having a temperature of 100°C, which was in a reflux state. The solution was continuously refluxed for 30 minutes and then left at room temperature to be cooled. The resultant gold colloidal solution was adjusted to be pH9 with a 0.2 M aqueous solution of potassium carbide. To the resultant solution, anti-hCG-α antibody solution was added and stirred for several minutes. Then, a 10% aqueous solution of BSA (bovine serum albumin) of pH9 was added thereto in such an amount as to provide a final concentration of 1%. Then, the mixture was stirred. Thus, an antibody-gold colloid complex (labeled antibody or the first substance group) as an indicator substance was obtained. The labeled antibody solution was centrifuged at 4°C for 50 minutes at 20,000 G so as to isolate the labeled antibody. The labeled antibody was suspended in a washing buffer solution (1% BSA-phosphate buffer). Then, the obtained substance was centrifuged so as to wash and isolate the labeled antibody. The labeled antibody was suspended in a washing buffer solution and filtered by a 0.8 µm filter. The amount of the labeled antibody was adjusted to 1/10 of the initial amount of the gold colloidal solution, and then the labeled antibody solution was stored at 4°C.

The labeled antibody solution was set in a solution injection apparatus, and applied to the nitrocellulose membrane **53** having the anti-hCG-β antibody (second substance group) immobilized thereon as the determination section **54.** The labeled antibody solution was applied to a position of the nitrocellulose membrane **53** which is far from the determination section **54.** Then, the nitrocellulose membrane **53** was dried. Thus, a reaction layer carrier (the nitrocellulose membrane **53**) having the indicator substance holding section **51** and the determination section **54** was produced.

The reaction layer carrier having the indicator substance holding section **51** thus produced was bonded to the support **55.** The sample application section formed of unwoven cloth and the water absorption section **56** formed of a glass fiber filter were provided on the reaction layer carrier. The obtained assembly was covered with a transparent tape (Nitto Denko; not shown) except for a part of the sample application section **52**, and then cut into a plurality of bio-devices each having a width of 0.5 cm.

Five bio-devices of each of eight types were produced using the results obtained with the position setting device **40.** More specifically, the eight types of bio-devices respectively had A:B ratios of 1:0.05, 1:0.25, 1:0.5, 1:0.75, 1:1, 1:1.05, 1:1.1, and 1:1.2. In other words, the eight types of bio-devices respectively had the B/A ratios of 5%, 25%, 50%, 75%, 100%, 105%, 110% and 120%.

The bio-devices thus produced were evaluated as follows.

### (d) Evaluation of the bio-devices

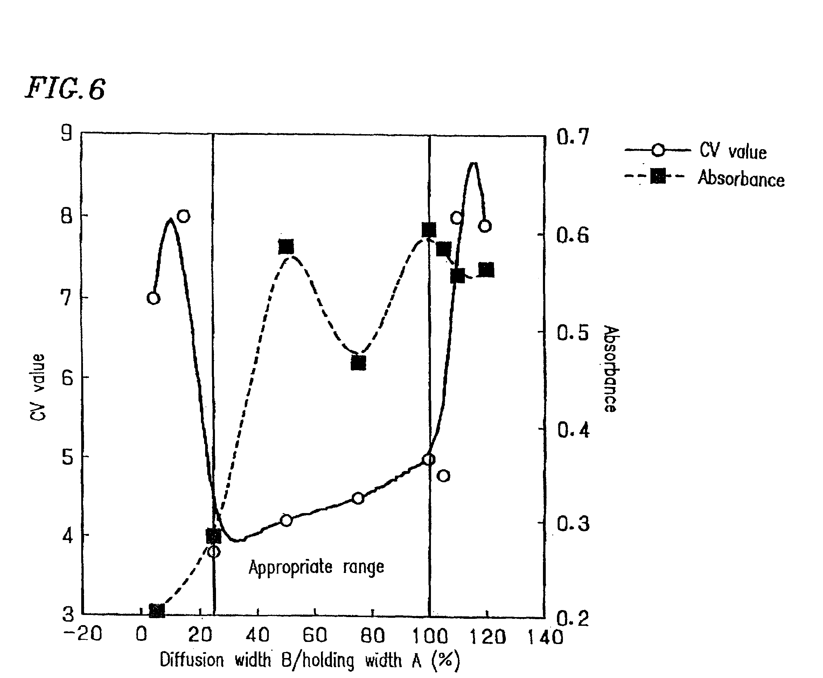

A urine sample containing 1000 U/l of hCG was applied to the sample application section **52** of each bio-device in an amount of about 40 µl. Five minutes later, the color of the determination section **54** caused by an antigen-antibody reaction was measured by reflection absorbance measurement. Specifically, the absorbance at 520 nm was measured using a reflection absorbance spectrometer (CS9300, Shimadzu Corporation). Figure **6** is a graph illustrating the resultant relationship among the B/A ratio, absorbance, and CV value. The CV value for each type of bio-device was obtained from the measurement values of five bio-devices. More specifically, the CV value is calculated by: the standard deviation of the measurement values/average of the measurement values × 100.

As can be appreciated from Figure **6,** when the B/A ratio was in the range of 25% to 100%, both the CV value and the absorbance were superb.

Next, three types of bio-devices having the B/Aratios of 5, 50 and 120% were tested in a similar manner using urine samples containing 0, 100, 1000 and 10000 U/l of hCG.

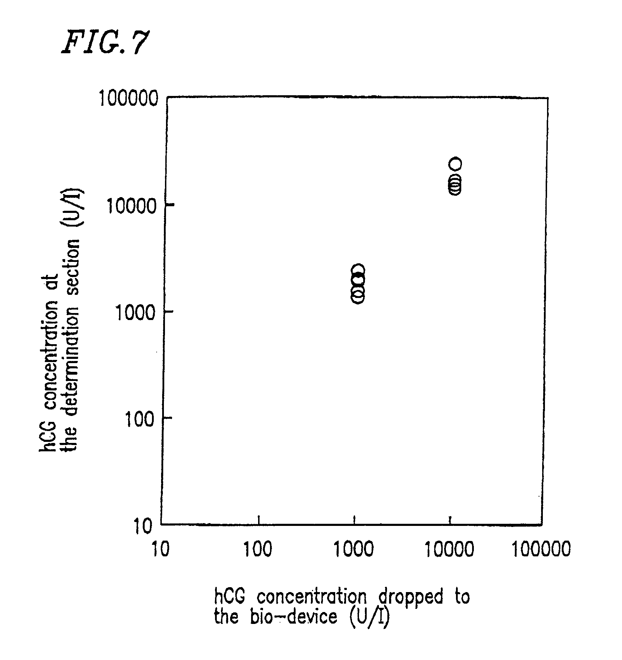

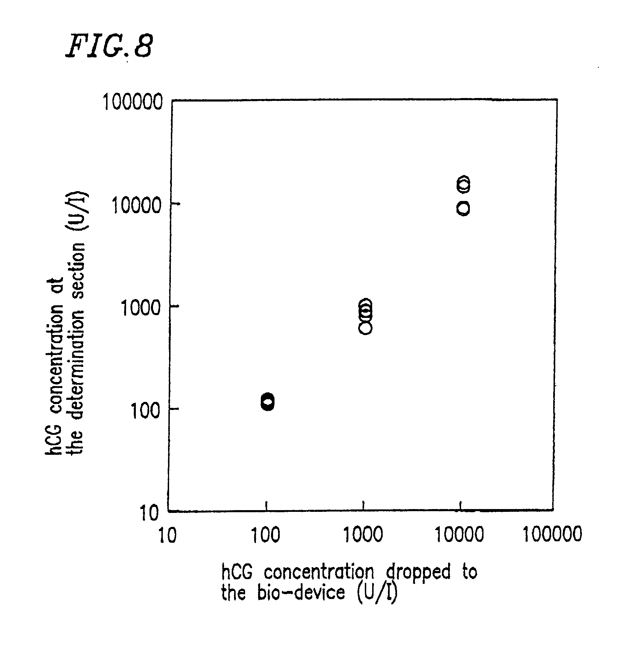

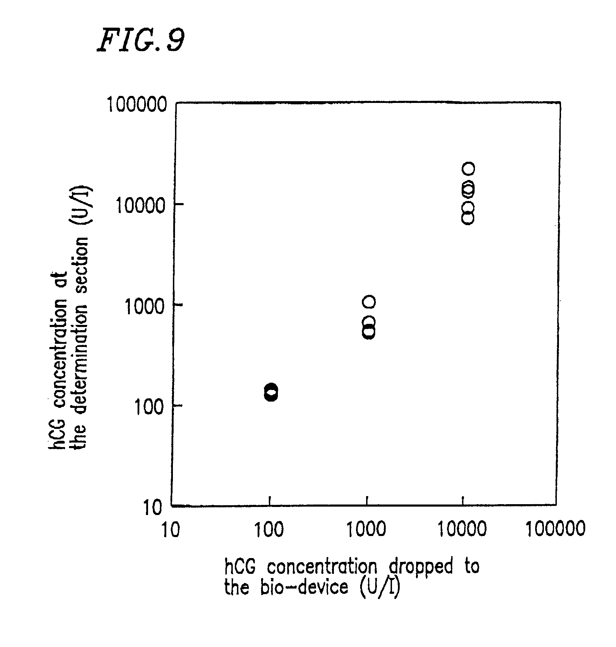

As described above, 40 µl of the urine samples were each applied to the sample application section **52** of each bio-device. Five minutes later, the color of the determination section **54** of each bio-device was measured using the reflection absorbance spectrometer and then the resultant color was subjected to an arithmetic operation. Specifically, the absorbance at 520 nm was measured, and substituted into a pre-prepared calibration curve illustrating the relationship between the hCG concentration and the absorbance. Figure **7** is a graph illustrating the results obtained with the bio-devices having the B/A ratio of 5%. Figure **8** is a graph illustrating the results obtained with the bio-devices having the B/A ratio of 50%. Figure **9** is a graph illustrating the results obtained with the bio-devices having the B/A ratio of 120%. In Figures **7**, **8** and **9**, the horizontal axis represents the hCG concentration of the urine sample delivered to the bio-device. The vertical axis represents the hCG concentration obtained by the above arithmetic operation based on the absorbance at 520 nm at the determination section **54**.

In an ideal condition, when the absorbance of a urine sample containing, for example, 1000 U/l of hCG is measured and the absorbance is substituted into the calibration curve, the hCG concentration should be 1000 U/l. In actuality, the value of the hCG concentration is deviated. The magnitude of the deviation represents the accuracy of the measurement.

As can be appreciated from Figure 7 regarding the bio-devices having the B/A ratio of 5%, the absorbance of the urine sample containing 100 U/l of hCG could not be measured. A possible reason is that the urine sample passed through the determination section **54** before the hCG contained in the urine sample is sufficiently captured by the immobilized anti-hCG-β antibody, and in a low concentration area of the hCG, measuring sensitivity was too low.

As can be appreciated from Figure **9** regarding the bio-devices having the B/A ratio of 120%, the measuring accuracy was especially low in a high concentration area of hCG. A possible reason is that since it took the urine sample an excessively long time to pass through the determination section **54**, disturbance in the flow flux of the urine sample, variance in the reaction or the like occurred.

As can be appreciated from Figure **8**, accurate and precise quantitative measurement results were obtained with the bio-devices having the B/A ratio of 50%.

### (Example 2)

Three types of bio-devices having substantially the same structure as those of Example 1 were prepared. In the three types of bio-devices, areas between the indicator substance holding section **51** and the determination section **54** were respectively 175 mm², 50 mm² and 5 mm². These bio-devices were subjected to substantially the same measurement as that of Example 1 using urine samples containing 0, 100, 1000 and 10000 U/l of hCG. The resultant absorbance was subjected to an arithmetic operation.

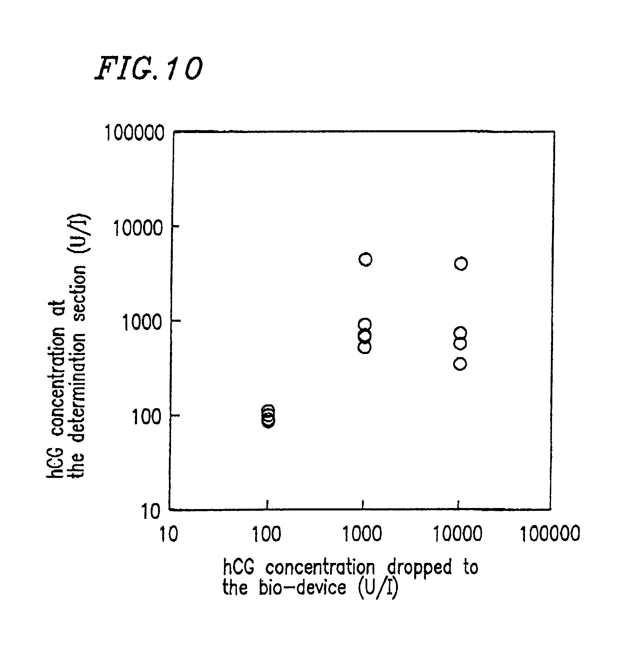

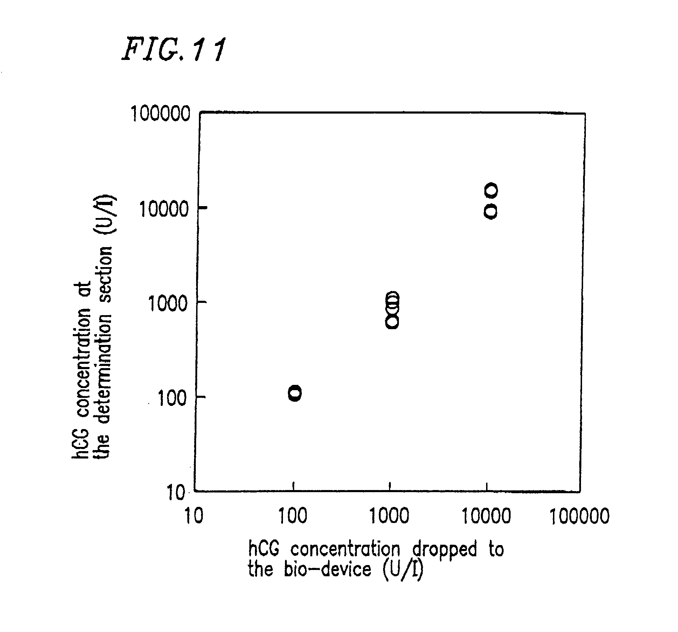

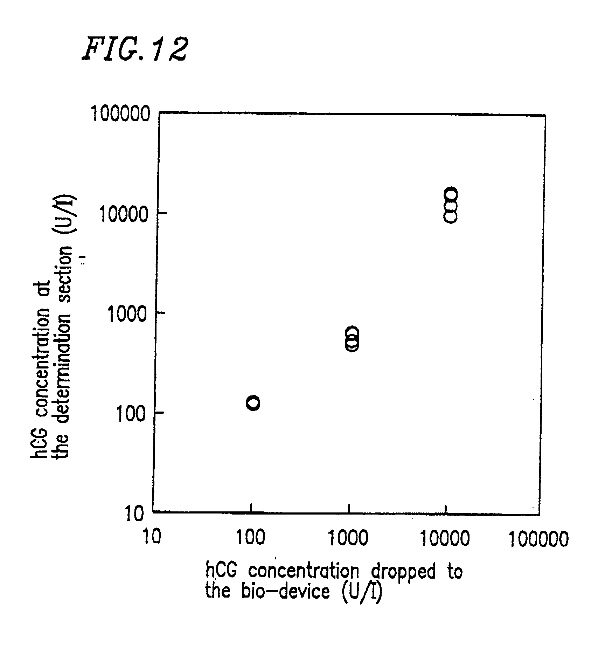

Figure **10** is a graph illustrating the results obtained with the bio-devices in which the area between the indicator substance holding section **51** and the determination section **54** was 175 mm². Figure **11** is a graph illustrating the results obtained with the bio-devices in which the area between the indicator substance holding section **51** and the determination section **54** was 50 mm². Figure **12** is a graph illustrating the results obtained with the bio-devices in which the area between the indicator substance holding section **51** and the determination section **54** was 5 mm². In Figures **10**, **11** and **12,** the horizontal axis represents the hCG concentration of the urine sample delivered to the bio-device. The vertical axis represents the hCG concentration obtained by the above arithmetic operation based on the absorbance at 520 nm at the determination section **54**.

As can be appreciated from Figure **10,** for the bio-devices in which the area between the indicator substance holding section **51** and the determination section **54** was 175 mm², the curve representing the relationship between the hCG concentration of the urine sample delivered to the bio-devices and the hCG concentration at the determination section **54** is not linear in a high concentration area of 10000 U/l. Additionally, the CV value exhibits a large variance of 10 to 100%, which means that this type of bio-devices do not provide accurate and precise quantitative measurement.

As can be appreciated from Figures **11** and **12**, when the bio-devices in which the area between the indicator substance holding section **51** and the determination section **54** was 50 mm² or 5 mm², the curve representing the relationship between the hCG concentration of the urine sample delivered to the bio-devices and the hCG concentration at the determination section **54** is linear up to a high concentration area of the hCG. The CV value of each type of bio-device exhibits 3 to 28%, which means that these types of bio-devices provide accurate and precise quantitative measurement.

The present invention optimizes the positional relationship between the indicator substance holding section and the determination section, and as a result, provides a bio-device realizing rapid, highly precise, and highly reproducible qualitative and quantitative measurement of a target substance in a liquid sample. The bio-device according to the present invention is suitable for POCT.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. A bio-device used for measuring a target substance included in a liquid sample, comprising:
a sample application section;
an indicator substance holding section; and
a determination section,
wherein:
the sample application section, the indicator substance holding section, and the determination section are located so that the liquid sample applied to the sample application section is transferred to the determination section via the indicator substance holding section,
at least the indicator substance holding section and the determination section are included in a single member,
the indicator substance holding section has a first substance group containing a substance specifically reacting with the target substance, wherein the first substance group is held so as to be capable of being eluted by the applied liquid sample, and
after the first substance group is eluted by the liquid sample applied to the sample application section, the first substance group flows as a mass having a leading end and a trailing end during flowing.

2. A bio-device used for measuring a target substance included in a liquid sample, comprising:
a sample application section;
an indicator substance holding section; and
a determination section,
wherein:
the sample application section, the indicator substance holding section, and the determination section are located so that the liquid sample applied to the sample application section is transferred to the determination section via the indicator substance holding section,
at least the indicator substance holding section and the determination section are included in a single member,
the indicator substance holding section has a first substance group containing a substance specifically reacting with the target substance, wherein the first substance group is held so as to be capable of being eluted by the applied liquid sample,
the determination section has a second substance group containing a substance specifically reacting with the target substance, the second substance group being in an immobilized state, and
in a process in which the first substance group contained in the indicator substance holding section is eluted by the action of the liquid sample applied to the sample application section and reaches the determination section together with the liquid sample while being diffused in a moving direction of the liquid sample, a holding width A of the indicator substance holding section in the moving direction of the liquid sample before the application of the liquid sample, and a diffusion width B, which is a width of the indicator substance, relative to the holding width A, in the moving direction of the liquid sample when a trailing end of the first substance group reaches the determination section, has a ratio A:B of 1:0.25 to 1:1.

3. A bio-device according to claim 1, wherein an area between the indicator substance holding section and the determination section is equal to or greater than 3 mm² and equal to or less than 150 mm².

4. A bio-device according to claim 1, wherein the sample application section, the indicator substance holding section, and the determination section are in a dry state before the liquid sample is applied.

5. A bio-device according to claim 1, wherein the liquid sample is a bodily fluid.

6. A bio-device according to claim 1, wherein the substance contained in the first substance group specifically reacting with the target substance is labeled with a coloring substance, a fluorescent substance, a phosphorescent substance, a light-emitting substance, an oxidoreductant, an enzyme, a nucleic acid, or an endoplasmic reticulum.

7. A bio-device according to claim 6, wherein the coloring substance is a gold colloidal particle.

8. A bio-device according to claim 2, wherein the first substance group includes a first antibody against the target substance, and the second substance group includes a second antibody against the target substance.

9. A bio-device according to claim 2, wherein the first substance group includes a first antibody and a second antibody against the target substance, the second antibody is labeled with biotin, and the second substance group includes avidin specifically reacting with biotin.

10. A bio-device according to claim 2, wherein the first substance group includes a first antibody and a second antibody against the target substance, the second antibody is labeled with a magnetic substance, and the second substance group includes a substance magnetically capturing the magnetic substance.

11. A bio-device according to claim 1, wherein the indicator substance holding section and the determination section are included in a porous member.

12. A bio-device according to claim 11, wherein the porous member is a nitrocellulose-based membrane.

13. A bio-device according to claim 11, wherein the sample application section is stacked on the porous member including the indicator substance holding section and the determination section.

14. A bio-device according to claim 11, further comprising a water absorption section provided on the porous member including the indicator substance holding section and the determination section, the water absorption section being opposite to the indicator substance holding section with respect to the determination section.

15. A bio-device according to claim 1, further comprising a member, which is non-permeable to the liquid sample, adhering to at least a portion of the sample application section, the indicator substance holding section and the determination section.

16. A quantitative measurement apparatus for measuring a target substance included in a liquid sample, the quantitative measurement apparatus comprising:
a bio-device according to claim 1; and
a measuring device for quantitatively measuring a physical or chemical signal obtained at a determination section of the bio-device.

17. A quantitative measurement method for measuring a target substance included in a liquid sample using the quantitative measurement apparatus according to claim 16, the quantitative measurement method comprising the steps of:
applying a prescribed amount of a liquid sample to a sample application section; and
quantitatively measuring a physical or chemical signal obtained at a determination section by a measuring device of the quantitative measurement apparatus.
